# FASCICULE DE BREVET EUROPEEN

(11) **EP 3 116 692 B1**
(45) Date de publication et mention de la délivrance du brevet: **18.11.2020**
(21) Numéro de dépôt: 15708540.8
(22) Date de dépôt: 10.03.2015
(51) Int. Cl.: B25J 9/16

(54) **METHODE D'INITIALISATION ET DE CONTROLE D'UNE INSTALLATION ROBOTISEE**
VERFAHREN ZUR INITIALISIERUNG UND STEUERUNG EINER ROBOTISCHEN VORRICHTUNG
METHOD FOR INITIALIZING AND CONTROLLING ROBOTIZED EQUIPMENT

(30) Priorité: 10.03.2014 FR 1451945
(43) Date de publication de la demande: 18.01.2017
(73) Titulaire: DiaMed GmbH, 1785 Cressier (CH)
(72) Inventeur: FAURE-VIDAL, Anaïs, F-42290 Sorbiers (FR); BERNAY, Sébastien, F-42670 Ecoche (FR); GAGNEPAIN, Cédric, 42300 Roanne (FR)
(74) Mandataire: Cabinet Beau de Loménie
(86) Numéro de dépôt international: PCT/EP2015/054924
(87) Numéro de publication internationale: WO 2015/135919

(56) Documents cités:
- WO-A1-2012/027541
- FR-A1- 2 987 896

## Description

### DOMAINE TECHNIQUE

Le présent exposé concerne une méthode d'initialisation et de contrôle d'une installation robotisée. Par "initialisation", on entend désigner une ou plusieurs opérations préalables au fonctionnement normal de l'installation, qui permettent d'autoriser et/ou d'améliorer ce fonctionnement.

Une telle méthode peut être utilisée, en particulier, pour une installation d'analyse médicale.

### ARRIERE PLAN

Les installations d'analyse médicale comprennent des appareils, appelés "automates d'analyse" ou "robots d'analyse", qui permettent d'automatiser certaines opérations qui seraient faites manuellement sinon. Par "analyse médicale", on entend désigner un processus consistant à traiter au moins un échantillon issu d'un être humain ou d'un animal. De tels échantillons sont, par exemple, des prélèvements de fluides corporels (sang, urine, lymphe, salive, etc.), de cellules, de tissus biologiques ou d'organe. Comme exemples d'analyses médicales, on peut citer les tests de groupe sanguin, les tests de recherche d'anticorps, les tests de détermination d'une compatibilité entre un donneur et un receveur, etc.

Des exemples d'installations d'analyse médicale sont décrits dans les documents US 6162399, JP 2010054232, EP 2145685, FR 2987896 et .... Ces installations utilisent toutes des robots de type cartésien. On appelle "robot de type cartésien" ou "robot cartésien", un robot ayant uniquement des articulations prismatiques pour le déplacement de son organe terminal (ou outil). En d'autres termes, dans la chaîne cinématique du robot, les trois degrés de liberté entre la base (ou socle) du robot et son organe terminal sont autorisés par au moins trois articulations prismatiques.

Les robots cartésiens, et plus particulièrement ceux qui ont un organe terminal non-articulé, ont pour particularité de simplifier la modélisation de l'espace de travail en ne tenant compte que de la position des objets dans l'espace, indépendamment de leur orientation. Le caractère réducteur de cette approche suppose que l'orientation des objets à gérer soit connue par avance, figée et reproductible sur chaque installation. Aussi, pour que le déplacement du robot cartésien par rapport aux objets à gérer soit précis, la mise en place de chaque installation doit être faite avec minutie pour que les objets à gérer soient orientés correctement par rapport au robot cartésien. Cette exigence complique sensiblement la mise en place de l'installation.

Aussi, il existe un besoin pour une solution qui permette de résoudre, au moins en partie, de tels inconvénients.

### PRESENTATION GENERALE

Le présent exposé concerne une méthode d'initialisation et de contrôle pour une installation robotisée comprenant un premier robot à quatre degrés de liberté minimum et un deuxième robot de type cartésien. Ci-après, ce deuxième robot est appelé indifféremment "deuxième robot" ou "robot cartésien" ou "deuxième robot de type cartésien".

Selon un mode de réalisation, la méthode comprend les étapes suivantes:
- on recueille à l'aide du premier robot des informations de localisation relatives à au moins un élément situé dans un espace de travail commun aux premier et deuxième robots; et
- on utilise les informations de localisation recueillies pour déplacer le deuxième robot dans ou autour de cet élément.

Par "informations de localisation", on entend désigner des informations relatives à la position et à l'orientation. De même, par "situation" d'un élément, on entend désigner la position et l'orientation de cet élément. Par "localiser", on entend désigner l'action consistant à recueillir des informations de localisation (i.e. à déterminer la situation d'un élément).

Le premier robot, du fait de sa liberté de mouvement plus grande que le robot cartésien, est plus "agile" que le robot cartésien. On utilise cet avantage du premier robot pour localiser un ou plusieurs éléments (i.e. des objets, des parties d'objets, des surfaces, etc.) situés dans un espace de travail commun aux premier et deuxième robots. On parlera ci-après d'étape de localisation. On utilise ensuite les informations de localisation recueillies pour le déplacement du robot cartésien dans cet espace de travail.

Il n'est donc pas nécessaire de positionner ces éléments par rapport au robot cartésien avec une grande précision lors de la mise en place de l'installation, puisque la situation de ces éléments peut être déterminée avec précision postérieurement à cette mise en place, lors de l'étape de localisation par le premier robot. Ceci permet de gagner un temps précieux lors de la mise en place de l'installation (cette mise en place intervient généralement lors du montage ou de la maintenance de l'installation). En outre, lorsqu'un élément est "mal" positionné par rapport à sa position théorique lors de la mise en place de l'installation, la "mauvaise" situation de cet élément est détectée pendant l'étape de localisation et il est tenu compte de cette "mauvaise" situation pour le déplacement du robot cartésien. Cet ajustement permet de réduire le risque de mauvais fonctionnement de l'installation.

On peut recueillir les informations de localisation en détectant l'élément à localiser au moyen d'un capteur, en particulier un palpeur, monté sur le premier robot. L'organe terminal du premier robot peut être équipé d'un tel capteur.

Les informations de localisation peuvent être recueillies de manière automatique par le premier robot. Par exemple, le premier robot est commandé par un programme et effectue automatiquement une certaine série de tâches visant à localiser ledit élément. Ce programme de commande peut être lancé après la mise en place de l'installation. De tels programmes de commande sont connus de l'art antérieur et certains robots sont mêmes vendus équipés de programmes de ce type.

L'élément à localiser peut être une zone inclinée par rapport aux plans de déplacement de l'organe terminal du robot cartésien, c'est-à-dire une zone formant un angle non nul par rapport aux trois plans définis par les trois paires d'axes des trois articulations prismatiques du robot cartésien. En particulier, un plan de déplacement de l'organe terminal du robot cartésien peut être horizontal et la zone à localiser peut être inclinée par rapport à l'horizontale. Par exemple, l'angle d'inclinaison de la zone à localiser par rapport à l'horizontale peut être compris entre 3 et 15° et, plus particulièrement, entre 5 et 10°. On notera qu'une telle zone est difficile à localiser correctement avec un robot cartésien équipé d'un palpeur du fait qu'il n'est pas possible de déplacer de manière précise le palpeur en suivant l'orientation de la zone. Il est donc avantageux de localiser cette zone avec le premier robot, ce dernier étant plus agile qu'un robot cartésien.

La zone inclinée peut correspondre au rebord périphérique d'une ouverture d'un récipient. Dans le domaine de l'analyse médicale, le fait de pouvoir localiser ce type de zone présente un intérêt particulier, comme expliqué dans la description détaillée ci-après.

Les informations de localisation recueillies par le premier robot sont généralement exprimées dans un premier système de coordonnées propre au premier robot et ne sont donc pas directement exploitables par le deuxième robot. Aussi, ces informations de localisation sont converties dans un deuxième système de coordonnées propre au deuxième robot avant d'être utilisées pour déplacer le deuxième robot. Différentes méthodes de conversion peuvent être utilisées. Par exemple, un gabarit peut être placé dans un espace de travail commun aux deux robots au moment de l'initialisation ou de la maintenance de l'installation, et le gabarit peut être localisé par le premier robot de manière à connaître les coordonnées (i.e. la position et l'orientation) du gabarit dans un repère du premier robot (par exemple le repère socle ou "world" du premier robot). La situation du même gabarit peut également être localisée par le deuxième robot de manière à connaître les coordonnées du gabarit dans un repère du deuxième robot (par exemple le repère socle ou "world" du deuxième robot). Il est alors possible d'en déduire une matrice de conversion entre les deux repères. Cette matrice de conversion peut ensuite être utilisée pour calculer, à partir des informations de localisation (i.e. des coordonnées) d'un élément dans le repère du premier robot, les informations de localisation de cet élément dans le repère du deuxième robot.

Outre les caractéristiques qui viennent d'être mentionnées plus haut, le dispositif/procédé proposé peut présenter une ou plusieurs des caractéristiques parmi les suivantes, considérées individuellement ou selon des combinaisons techniquement possibles:
- le premier robot possède six degrés de liberté et au moins deux articulations rotoïdes,
- l'organe terminal du deuxième robot est non-articulé par rapport au reste du deuxième robot (i.e. par rapport au porteur du robot).

Les caractéristiques et avantages précités, ainsi que d'autres, apparaîtront à la lecture de la description détaillée qui suit, d'un exemple d'application de la méthode proposée. Cette description détaillée fait référence aux dessins annexés.

### BREVE DESCRIPTION DES DESSINS

Les dessins annexés sont schématiques et ne sont pas à l'échelle, ils visent avant tout à illustrer les principes de l'invention.

Sur ces dessins, d'une figure (FIG) à l'autre, des éléments (ou parties d'élément) identiques sont repérés par les mêmes signes de référence.
La FIG 1 représente un exemple d'installation d'analyse médicale robotisée.
La FIG 2 représente un exemple de récipient utilisé dans l'installation de la FIG 1.
La FIG 3 est une vue de dessus du récipient de la FIG 2 suivant la flèche III.
La FIG 4 représente en demi-coupe axiale, suivant le plan IV-IV, le récipient des FIGS 2 et 3.
La FIG 5 est une vue semblable à celle de la FIG 4, représentant une étape de détection du fond du récipient.
La FIG 6 est une vue semblable à celle de la FIG 4, représentant une étape de déplacement de l'organe terminal du robot cartésien de la FIG 1 dans le récipient.

### DESCRIPTION DETAILLEE D'EXEMPLE(S)

Un exemple de réalisation est décrit en détail ci-après, en référence aux dessins annexés. Cet exemple illustre les caractéristiques et les avantages de l'invention. Il est toutefois rappelé que l'invention ne se limite pas à cet exemple.

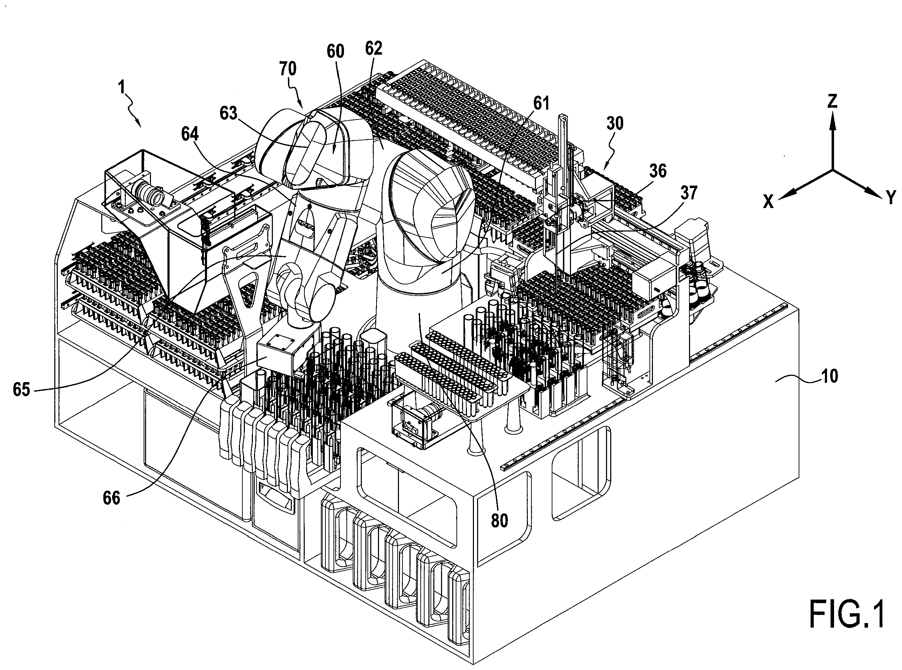

La FIG 1 représente une installation robotisée 1 permettant d'effectuer des analyses médicales. Cette installation 1 comprend un châssis 10 supportant un premier robot 70 à quatre degrés de liberté minimum et un deuxième robot 30 de type cartésien, ainsi qu'une pluralité de récipients adaptés pour recevoir, en particulier, des échantillons à analyser et/ou des réactifs avec lesquels les échantillons réagissent.

Le premier robot 70 est situé sensiblement au centre de l'installation 1 et est muni d'un bras poly-articulé 60. Dans l'exemple, le bras 60 comprend un premier segment de bras 61 s'étendant depuis un socle horizontal 80 fixé au châssis 10 et situé sensiblement au centre de l'installation 1. Le premier segment de bras 61 est monté pivotant par rapport au socle 80, autour d'un premier axe A1 sensiblement vertical. Un deuxième segment de bras 62, est relié au premier segment 61 et monté pivotant par rapport à celui-ci, autour d'un deuxième axe A2 perpendiculaire au premier axe A1. Un troisième segment de bras 63 est relié au deuxième segment 62 et monté pivotant par rapport à celui-ci, autour d'un troisième axe A3 parallèle à A2. Un quatrième segment de bras 64 est relié au troisième segment de bras 63 et monté pivotant par rapport à celui-ci autour d'un quatrième axe A4 perpendiculaire au troisième axe A3. Un cinquième segment de bras 65 est relié au quatrième segment de bras 64 en étant monté pivotant par rapport à celui-ci autour d'un cinquième axe A5 perpendiculaire au quatrième axe A4. Enfin, le bras 60 est terminé par un sixième segment de bras ou organe terminal 66 relié à l'extrémité du cinquième bras 65 opposée au quatrième bras 64. L'organe terminal 66 est monté pivotant par rapport au cinquième bras 65 autour d'un sixième axe A6 perpendiculaire au cinquième axe A5. Grâce aux six articulations rotoïdes d'axes de rotation respectifs A1 à A6, l'organe terminal 66 peut atteindre tous les éléments répartis à 360° autour de lui, à des hauteurs différentes et selon des orientations différentes. On notera que le premier robot 70 pourrait atteindre une liberté de mouvement équivalente avec un agencement d'articulations différent.

Le deuxième robot 30 est un robot de type cartésien ayant des articulations prismatiques pour le déplacement de son organe terminal (ou outil) 36. Ces articulations permettent de déplacer l'organe terminal suivant les trois axes X, Y et Z d'un repère cartésien. Le plan XY contenant les axes X et Y de ce repère est un plan sensiblement horizontal. L'organe terminal 36 de ce robot est monté fixe (i.e. n'est pas articulé) par rapport au reste du robot (i.e. par rapport au porteur du robot).

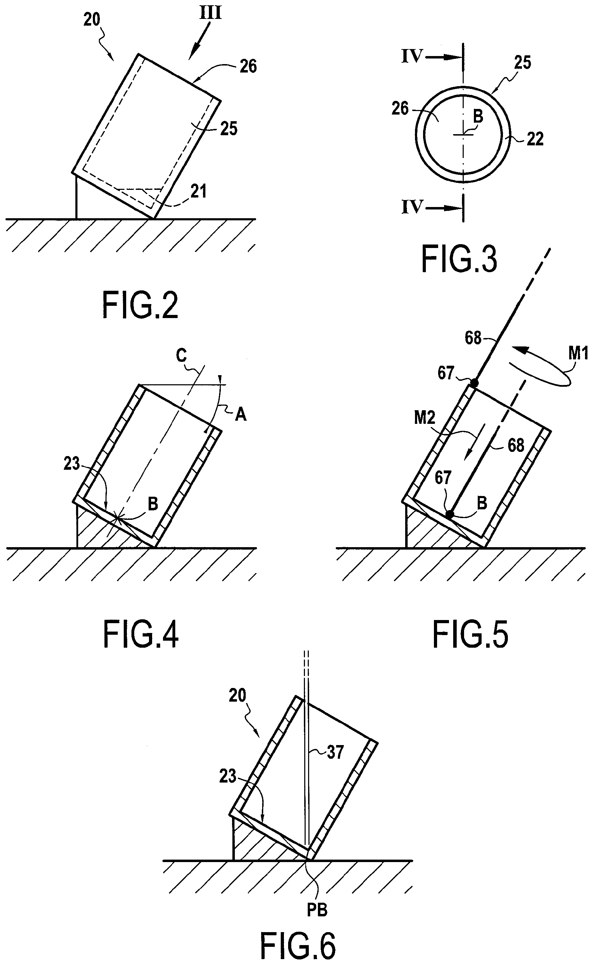

Dans cette installation, le robot cartésien 30 est notamment utilisé pour prélever une certaine quantité de réactif 21 à l'intérieur d'un récipient 20, représenté sur la FIG 2. Pour ce faire, l'organe terminal 36 du robot cartésien 30 est équipé d'un dispositif de prélèvement comme une pipette 37 permettant d'aspirer une quantité déterminée de réactif 21 à chaque prélèvement.

Dans le domaine de l'analyse médicale, certains réactifs sont très onéreux. Aussi, lorsqu'un tel réactif est contenu dans un récipient, on chercher à pouvoir prélever la totalité (ou la quasi-totalité) du réactif présent dans le récipient (i.e. à éviter de laisser une quantité de réactif non-utilisée dans le récipient), pour éviter de gâcher la moindre quantité de réactif.

Dans l'exemple des figures, le récipient 20 a la forme d'un cylindre de révolution d'axe C. Il comprend une paroi latérale cylindrique 25 s'étendant entre une paroi de fond 23 et une ouverture 26 opposée à la paroi de fond 23. L'extrémité de la paroi latérale 25 définit le bord 22 de l'ouverture 26. Le bord 22 est contenu dans un plan incliné par rapport au plan XY horizontal. Cette inclinaison est représentée par l'angle A sur la FIG 4. Le centre de la paroi de fond 23 est noté B. L'axe C passe par le centre B.

Le récipient 20 se situe dans un espace de travail commun aux premier et deuxième robots 70, 30 de l'installation 1.

Pour pouvoir prélever par aspiration à l'aide de la pipette 37 la totalité (ou quasi-totalité) du réactif 21 présent dans le récipient 20, le récipient 20 est incliné par rapport à l'horizontale, comme représenté sur les figures. Ainsi, le point le plus bas PB du récipient est un point situé à la périphérie de la paroi de fond 23 et le réactif 21 se répartit autour de ce point PB. En amenant le bout de la pipette 37 au niveau du point PB (voir FIG. 6), on peut aspirer la totalité (ou quasi-totalité) du réactif 21 présent dans le récipient 20. On comprend donc qu'il est important de pouvoir amener le bout de la pipette 37 le plus précisément possible au niveau du point PB et que tout décalage du bout de la pipette par rapport à ce point PB conduit à une perte de réactif. Pour ce faire, il faut que le robot cartésien 30 qui porte la pipette « connaisse » exactement la position du point PB.

Pour localiser le point PB, on met en place un récipient 20 (généralement un récipient vide) et on détecte le bord 22 de l'ouverture 26 au moyen d'un palpeur 67 (e.g. un palpeur piézo-électrique) monté au bout d'une tige 68 elle-même montée sur l'organe terminal 66 du premier robot 70. Cette opération ne présente pas de difficulté du fait des capacités de déplacement du premier robot 70. En particulier, l'organe terminal 66 peut être incliné de sorte que le palpeur 67 suive le bord 22 en se déplaçant dans le plan contenant le bord 22. Cette opération est représentée schématiquement sur la FIG 5 par la flèche M1. Le bord 22 est un exemple de zone inclinée par rapport aux plans de déplacement de l'organe terminal 36 du deuxième robot 30.

Une fois le bord 22 détecté et localisé, il est possible de déduire par calcul la situation de l'axe C. On amène alors le palpeur 67 jusqu'au centre B de la paroi de fond 23 en suivant l'axe C. Cette opération est représentée schématiquement sur la FIG 5 par la flèche M2. Selon une alternative, au lieu de palper directement le fond du récipient 20, on peut palper le fond d'un logement destiné à accueillir le récipient 20 et en déduire par calcul (i.e. en tenant compte de l'épaisseur de la paroi de fond 23) la situation du centre B du fond du récipient. De telles opérations ne présentent pas de difficulté du fait des capacités de déplacement du premier robot 70 (il serait en revanche impossible de réaliser cette opération à l'aide du premier robot 30). En connaissant la situation du bord 22 et du centre B de la paroi de fond 23, on peut déduire par calcul la situation du point le plus bas PB.

Une fois la situation du point le plus bas PB connue dans un repère (par exemple, le repère socle) du premier robot 70, on détermine par calcul à l'aide d'une matrice de conversion la situation du point le plus bas PB dans un repère (par exemple le repère socle) du deuxième robot 30. A l'aide de cette information, le bout de la pipette 37 peut être déplacé avec précision jusqu'au point PB et la totalité (ou quasi-totalité) du réactif 21 peut être prélevée.

Bien entendu, il ne s'agit ici que d'un exemple d'application de la méthode proposée et cet exemple est donné à titre illustratif et non limitatif. En particulier, la méthode peut être appliquée à d'autres types et d'autres formes de récipient et la position du point le plus bas du récipient pourrait être calculée selon d'autres méthodes s'appuyant sur la localisation d'autres zones du récipient ou sur la localisation de certaines zones d'un support du récipient. Plus généralement, au vu du présent exposé, une personne du métier pourrait modifier les modes ou exemples de réalisation donnés, ou en envisager d'autres, tout en restant dans la portée de l'invention.

De plus, les différentes caractéristiques de ces modes ou exemples de réalisation peuvent être utilisées seules ou être combinées entre elles. Lorsqu'elles sont combinées, ces caractéristiques peuvent l'être comme décrit ci-dessus ou différemment, l'invention ne se limitant pas aux combinaisons spécifiques décrites dans le présent exposé. En particulier, sauf précision contraire, une caractéristique décrite en relation avec un mode ou exemple de réalisation peut être appliquée de manière analogue à un autre mode ou exemple de réalisation.

## Revendications

1. Méthode d'initialisation et de contrôle d'une installation robotisée comprenant un premier robot (70) à quatre degrés de liberté minimum et un deuxième robot (30) de type cartésien, la méthode comprenant les étapes suivantes:
- on recueille à l'aide du premier robot (70) des informations de localisation relatives à au moins un élément située dans un espace de travail commun aux premier et deuxième robots; et
- on utilise les informations de localisation recueillies pour déplacer le deuxième robot (30) dans ou autour de cet élément.

2. Méthode selon la revendication 1, dans laquelle on recueille les informations de localisation en détectant l'élément au moyen d'un capteur, en particulier un palpeur (67), monté sur le premier robot (70).

3. Méthode selon la revendication 1 ou 2, dans laquelle les informations de localisation sont recueillies de manière automatique par le premier robot (70).

4. Méthode selon l'une quelconque des revendications 1 à 3, dans laquelle ledit élément est une zone inclinée par rapport aux plans de déplacement de l'organe terminal (36) du deuxième robot (30).

5. Méthode selon l'une quelconque des revendications 1 à 4, dans laquelle le premier robot (70) possède six degrés de liberté et au moins deux articulations rotoïdes.

6. Méthode selon l'une quelconque des revendications 1 à 5, dans laquelle les informations de localisation recueillies sont exprimées dans un premier système de coordonnées propre au premier robot (70), ces informations de localisation étant converties dans un deuxième système de coordonnées propre au deuxième robot (30) avant d'être utilisées pour déplacer le deuxième robot.

7. Méthode selon l'une quelconque des revendications 1 à 6, dans laquelle l'installation robotisée (1) est une installation d'analyse médicale.

8. Méthode selon l'une quelconque des revendications 1 à 7, dans laquelle la zone inclinée correspond au bord (22) d'une ouverture (26) d'un récipient (20).

## Patentansprüche

1. Verfahren zum Initialisieren und Steuern einer Roboteranlage, umfassend einen ersten Roboter (70) mit mindestens vier Freiheitsgraden und einen zweiten Roboter (30) vom Typ des kartesischen Roboters, wobei das Verfahren die folgenden Schritte aufweist:
- mit Hilfe des ersten Roboters (70) werden Positionsinformationen gesammelt, die sich auf mindestens ein Element beziehen, das sich in einem Arbeitsbereich befindet, der dem ersten und dem zweiten Roboter gemeinsam ist, und
- die gesammelten Positionsinformationen werden verwendet, um den zweiten Roboter (30) in oder um dieses Element zu bewegen.

2. Verfahren nach Anspruch 1, wobei die Positionsinformationen gesammelt werden, indem das Element mittels eines Sensors, insbesondere eines Tasters (67) erfasst wird, der auf dem ersten Roboter (70) befestigt ist.

3. Verfahren nach Anspruch 1 oder 2, wobei die Positionsinformationen von dem ersten Roboter (70) automatisch gesammelt werden.

4. Verfahren nach einem der Ansprüche 1 bis 3, wobei das Element ein Bereich ist, der in Bezug auf die Bewegungsebenen des Endorgans (36) des zweiten Roboters (30) geneigt ist.

5. Verfahren nach einem der Ansprüche 1 bis 4, wobei der erste Roboter (70) sieben Freiheitsgrade und mindestens zwei Drehgelenke aufweist.

6. Verfahren nach einem der Ansprüche 1 bis 5, wobei die gesammelten Positionsinformationen in einem ersten Koordinatensystem, das dem ersten Roboter (70) eigen ist, ausgedrückt werden, wobei diese Positionsinformationen in ein zweites Koordinatensystem, das dem zweiten Roboter (30) eigen ist, umgewandelt werden, bevor sie verwendet werden, um den zweiten Roboter zu bewegen.

7. Verfahren nach einem der Ansprüche 1 bis 6, wobei die Roboteranlage (1) eine medizinische Analyseeinrichtung ist.

8. Verfahren nach einem der Ansprüche 1 bis 7, wobei der geneigte Bereich dem Rand (22) einer Öffnung (26) eines Behälters (20) entspricht.

## Claims

1. A method of initializing and controlling a robotic installation comprising a first robot (70) having a minimum of four degrees of freedom and a second robot (30) that is of Cartesian type, the method comprising the following steps:
• using the first robot (70) to collect location information relating to at least one element situated in a workspace common to the first and second robots; and
• using the collected location information to move the second robot (30) in or around the element.

2. A method according to claim 1, wherein the location information is collected by detecting the elements by means of a sensor, in particular a feeler (67), that is mounted on the first robot (70).

3. A method according to claim 1 or claim 2, wherein the location information is collected automatically by the first robot (70).

4. A method according to any one of claims 1 to 3, wherein said element is an inclined zone sloping relative to the travel planes of the terminal member (36) of the second robot (30).

5. A method according to any one of claims 1 to 4, wherein the first robot (70) possesses six degrees of freedom and at least two rotoid joints.

6. A method according to any one of claims 1 to 5, wherein the collected location information is expressed in a first coordinate system specific to the first robot (70), this location information being converted into a second coordinate system specific to the second robot (30) prior to being used for moving the second robot.

7. A method according to any one of claims 1 to 6, wherein the robotic installation (1) is a medical analysis installation.

8. A method according to any one of claims 1 to 7, wherein the inclined zone corresponds to the edge (22) of an opening (26) of a container (20).
